(19) Europäisches Patentamt
European Patent Office
Office européen des brevets

(11) **EP 2 164 462 B1**

(12) **EUROPÄISCHE PATENTSCHRIFT**

(45) Veröffentlichungstag und Bekanntmachung des Hinweises auf die Patenterteilung:
**31.05.2017 Patentblatt 2017/22**

(21) Anmeldenummer: **08760389.0**

(22) Anmeldetag: **03.06.2008**

(51) Int Cl.:
*A61K 9/00* (2006.01)   *A61K 9/20* (2006.01)
*A61K 9/16* (2006.01)

(86) Internationale Anmeldenummer:
**PCT/EP2008/056805**

(87) Internationale Veröffentlichungsnummer:
**WO 2008/148742 (11.12.2008 Gazette 2008/50)**

(54) **PHARMAZEUTISCHE FORMULIERUNG FÜR DIE HERSTELLUNG VON SCHNELL ZERFALLENDEN TABLETTEN**

PHARMACEUTICAL FORMULATION FOR THE PRODUCTION OF RAPIDLY DISINTEGRATING TABLETS

PRÉPARATION PHARMACEUTIQUE POUR PRODUIRE DES COMPRIMÉS À DÉLITEMENT RAPIDE

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **06.06.2007 EP 07109722**

(43) Veröffentlichungstag der Anmeldung:
**24.03.2010 Patentblatt 2010/12**

(73) Patentinhaber: **BASF SE**
**67056 Ludwigshafen am Rhein (DE)**

(72) Erfinder:
• KOLTER, Karl
  67117 Limburgerhof (DE)
• SCHÖNHERR, Michael
  67227 Frankenthal (DE)
• GEBERT, Silke
  67269 Grünstadt (DE)
• MEYER-BÖHM, Kathrin
  90537 Feucht (DE)
• MASCHKE, Angelika
  68159 Mannheim (DE)

(74) Vertreter: **BASF IP Association**
**BASF SE**
**G-FLP-C006**
**67056 Ludwigshafen (DE)**

(56) Entgegenhaltungen:
WO-A-98/22094       WO-A-03/041683
WO-A-2007/071581    US-A1- 2002 071 864
US-A1- 2005 244 343   US-A1- 2005 244 492

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft pharmazeutische Formulierungen in Form von Agglomeraten für die Herstellung von schnell zerfallenden Tabletten, enthaltend neben mindestens einem Wirkstoff Zucker oder Zuckeralkohole, Sprengmittel und wasserunlösliche Polymere.

Im Mund schnell zerfallende und/oder sich schnell auflösende Tabletten gewinnen für die orale Applikation von Arzneistoffen immer größere Bedeutung. Solche Tabletten müssen innerhalb kurzer Zeit, am besten innerhalb von 30 Sekunden in der Mundhöhle zerfallen, angenehm schmecken und dürfen kein sandiges Gefühl hinterlassen. Ferner sollen sie einfach herstellbar sein, wobei die Direkttablettierung erhebliche Vorteile gegenüber der Feuchtgranulation bietet, und eine hohe mechanische Festigkeit besitzen, damit sie Verpackungsprozeduren, Transporte und auch das Herausdrücken aus Verpackungen unbeschadet überstehen.

Die bisher beschriebenen Produkte und Verfahren erfüllen diese Anforderungen nicht oder nur sehr unzureichend.

Schnell zerfallende Tabletten bestehen häufig aus Zucker und Zuckeralkoholen, Brausesystemen, mikrokristalliner Cellulose und anderen nicht wasserlöslichen Füllstoffen Calciumhydrogenphosphat, Cellulose-Derivaten, Maisstärke, oder Polypeptiden. Weiterhin kommen wasserlösliche Polymere, übliche Sprengmittel (quervernetztes PVP, Na- und Ca-Salz der quervernetzten Carboxymethylcellulose, Na-Salz der Carboxymethylstärke, niedrigsubstituierte Hydroxypropylcellulose L-HPC) und im wesentlichen anorganische wasserunlösliche Bestandteile (Kieselsäuren, Silikate, anorganische Pigmente) zum Einsatz. Weiterhin können die Tabletten auch Tenside enthalten.

In der WO 2003/051338 ist eine direkttablettierbare und gut verpressbare Hilfsstoffformulierung, welche Mannit und Sorbit enthält, beschrieben. Zunächst wird durch Lösen von Mannit und Sorbit in Wasser und anschließender Sprühtrocknung (gewöhnliche Sprühtrocknung und SBD-Verfahren) eine Hilfsstoffvormischung hergestellt. Dieser coprozessierten Mischung kann zusätzlich Mannit zugesetzt werden. Tabletten, welche zusätzlich Sprengmittel, Trennmittel, Pigment und einen Wirkstoff enthalten, sollen innerhalb von 60 Sekunden in der Mundhöhle zerfallen.

In der US 2002/071864 A1 wird eine Tablette beschrieben, welche innerhalb von 60 Sekunden in der Mundhöhle zerfällt, und hauptsächlich aus einer physikalischen Mischung von sprühgetrocknetem Mannit und einem grobkörnigen quervernetztem Polyvinylpyrrolidon sowie einer begrenzten Auswahl an Wirkstoffen formuliert ist. Diese Tabletten besitzen eine Bruchfestigkeit von ca. 40N und erzeugen ein unangenehmes, sandiges Mundgefühl.

Gemäß der US 6,696,085 B2 soll ein Methacrylsäure-Copolymer Typ C als Zerfallsmittel eingesetzt werden. Das Methacrylsäure-Copolymer Typ C ist ein magensaftresistentes Polymer, welches im sauren pH-Bereich nicht löslich ist, im pH-Bereich von 7, wie er in der Mundhöhle vorliegt, aber wasserlöslich ist. Die Tabletten weisen neben einer niedrigen Bruchfestigkeit (<20N) eine hohe Friabilität (>7%) auf und beinhalten einen hohen Anteil im Bereich von 15Gew.-% eines grobkörnigen Sprengmittels. Sie besitzen folglich eine niedrige mechanische Festigkeit und erzeugen aufgrund des hohen Anteils an grobkörnigem Sprengmittel ein unangenehmes, sandiges Mundgefühl.

EP 0839526 A2 beschreibt eine pharmazeutische Darreichungsform bestehend aus einem Wirkstoff, Erythrit, kristalliner Cellulose und einem Sprengmittel. Weiterhin wird Mannit eingearbeitet und als Sprengmittel quervernetztes Polyvinylpyrrolidon verwendet, sodass eine physikalische Mischung entsteht. Die Tabletten sollen innerhalb von 60 Sekunden in der Mundhöhle zerfallen.

In der Anmeldung JP 2004-265216 wird eine im Mund innerhalb von 60 Sekunden zerfallende Tablette, bestehend aus einem Wirkstoff, einem wasserlöslichen Polyvinylalkohol-Polyethylenglykol-Copolymer, Zucker/Zuckeralkohol (Mannit) und Sprengmittel, beschrieben.

In der WO 2003/041683 werden schnell auflösende Mundtabletten beschrieben enthaltend 30-90 Gew-% Mannitol, 3-15 Gew.-% eines Sprengmittels ausgewählt aus Croscarmellose, Crospovidon, vorzugsweise weniger als 10 Gew-% eines wasserunlöslichen Polymers wie z. B. Ethylcellulose und ein viskositätserhöhendes oder gelbildendes Polymer wie z.B. HPMC, Alginat etc..

Sowohl die US 2005244343 als auch die US 2005244492 beschreiben schnell auflösende Mundtabletten enthaltend 27-66,25 Gew.-% eines Zuckeralkohols wie z.B. Mannitol oder Erythriol, 5-10 Gew.-% eines Sprengmittels ausgewählt aus Stärkederivaten, Crospovidon, Croscarmellose, sowie Xanthan als viskositätserhöhendem Polymer. Sie unterscheiden sich von der vorliegenden Anmeldung dadurch, dass keine wasserunlöslichen filmbildenden Polymere verwendet werden, sondern Silikate bzw. Titandioxid.

Aufgabe der vorliegenden Erfindung war es, im Mund schnell zerfallende Tabletten zu finden, die ein angenehmes Mundgefühl hinterlassen, mechanisch sehr stabil sind und eine hohe Gehaltseinheitlichkeit aufweisen.

Demgemäß wurde eine pharmazeutische Zubereitung für die Herstellung von im Mund schnell zerfallenden Tabletten gefunden, welche aus Agglomeraten, enthaltend

   A) einen Anteil an Hilfsstoffen aus

      a) 60 - 97 Gew.-% mindestens eines Zuckers oder Zuckeralkohols oder Mischungen davon,

b) 1 - 25 Gew.-% einesSprengmittels,
c) 1 - 15 Gew.-% von wasserunlöslichen Polymeren,
d) 0 - 15 Gew.-% wasserlöslichen Polymeren, und
e) 0 - 15 Gew.-% weiterer pharmazeutisch üblichen Hilfsstoffen,

wobei die Summe der Komponenten a) bis e) 100 Gew.-% beträgt, und

B) mindestens einen Wirkstoff,

besteht.

Weiterhin wurden Verfahren zur Herstellung solcher Agglomerate gefunden.

Weiterhin wurden im Mund schnell zerfallende Tabletten, enthaltend solche Zubereitungen, gefunden. Die Tabletten zerfallen im Mund oder in wässrigem Milieu innerhalb von 60 Sekunden, bevorzugt innerhalb von 30 Sekunden, besonders bevorzugt innerhalb von 20 Sekunden. "Die Tabletten weisen bei 37 °C in Phospatpuffer, pH 7,2, eine Zerfallszeit von < 60 Sekunden auf. Die Zerfallszeit wird im Zerfallstester nach USP oder Pharm. Eur. bestimmt."

Der Hilfsstoffanteil A) setzt sich im Einzelnen wie folgt zusammen:

Die pharmazeutischen Zubereitungen enthalten als Komponente a) 60 bis 97 Gew.-%, bevorzugt 70 bis 95 Gew.-%, besonders bevorzugt 75 bis 93 Gew.-% eines Zuckers, Zuckeralkohols oder Mischungen davon. Als Zucker oder Zuckeralkohole eignen sich Trehalose, Mannit, Erythrit, Isomalt, Maltit, Lactit, Xylit, Sorbit. Die Zucker- oder Zuckeralkoholkomponenten sind bevorzugt feinteilig, mit mittleren Teilchengrößen von 5 bis 100 $\mu$m. Gewünschtenfalls können die Teilchengrößen durch Mahlen eingestellt werden. Bevorzugte Teilchengrößen liegen bei 30 bis 50 $\mu$m. Es kann sich aber auch empfehlen Teilchengrößen kleiner 30 $\mu$m zu verwenden. Ebenso kann es sich empfehlen, Zucker beziehungsweise Zuckeralkohole einzusetzen, die Mischungen von Fraktionen mit unterschiedlicher Partikelgröße enthalten, bespielsweise Mischungen aus 30 bis 70 Gew.-% einer Korngrößenfraktion mit einer mittleren Teilchengröße von < 30 $\mu$m und 30 bis 70 Gew.-% einer Korngrößenfraktion mit einer mittleren Teilchgröße von 30 bis 50 $\mu$m. Bevorzugt werden Mannit, Erythrit oder Mischungen davon eingesetzt.

Als Komponente b) werden Sprengmittel in Mengen von 1 bis 25 Gew.-%, bevorzugt, 2 bis 15 Gew.-%, besonders bevorzugt 3 bis 10 Gew.-%, eingesetzt. Die Sprengmittel sind bevorzugt ausgewählt aus der Gruppe bestehend aus quervernetzten Polyvinylpyrrolidonen, Croscarmellose, Natriumcarboxymethylstärke und L-Hydroxypropylcellulose. Unter Croscarmellose werden erfindungsgemäß die Natrium- und/oder Calciumsalze der quervernetzten Carboxymethycellulose verstanden. Bevorzugte L-Hydroxypropylcellulosen weisen 5 bis 16% Hydroxypropoxy-Gruppen auf.

Besonders bevorzugt sind quervernetzte Polyvinylpyrrolidone. Solche quervernetzten Polyvinylpyrrolidone sind wasserunlöslich, aber nicht filmbildend. Das quervernetzte Polyvinylpyrrolidon kann eine mittlere Teilchengröße von 2 bis 60 $\mu$m, bevorzugt kleiner 50 $\mu$m, besonders bevorzugt kleiner 30 $\mu$m aufweisen. Ganz besonders bevorzugt sind quervernetzte Polyvinylpyrrolidone mit einer Hydratationskapazität von größer 6,5 g/g. Hierbei erfolgt die Bestimmung der Hydratationskapazität nach folgender Methode: 2 g Polymer werden in ein Zentrifugenglas eingewogen und mit 40ml Wasser 15 Minuten ausquellen lassen. Anschließend wird 15 Minuten bei 2000U/Min zentrifugiert und die überstehende Flüssigkeit so vollständig wie möglich abgegossen.

$$\text{Hydratationskapazität} = \frac{\text{Auswaage-Tara}}{\text{Einwaage}}$$

Die hohe Hydratationskapazität des quervernetzten Polyvinylpyrrolidons führt in der Formulierung zu einem sehr schnellen Zerfall und ergibt ein besonders weiches Mundgefühl.

Als Komponente c) werden wasserunlösliche Polymere eingesetzt in Mengen von 1 bis 15 Gew.-%, bevorzugt 1 bis 10 Gew.-%, eingesetzt. Dabei handelt es sich um Polymere. Bevorzugt sind Polymere, die im pH-Bereich von 1 bis 14 unlöslich sind, also eine bei jedem pH-Wert pH-unabhängige Wasserunlöslichkeit aufweisen. Weiterhin eignen sich aber auch Polymere, die bei jedem pH-Wert im pH-Bereich von 6 bis 14 wasserunlöslich sind.

Die Polymere sollen filmbildende Polymere sein. Filmbildend bedeutet in diesem Zusammenhang, dass die Polymere in wässriger Dispersion eine Mindestfilmbildetemperatur von -20 bis +150 °C, bevorzugt 0 bis 100 °C aufweisen.

Geeignete Polymere sind Polyvinylacetat, Ethylcellulose, Methylmethacrylat-Ethylacrylat-Copolymere, Ethylacrylat-Methylmethacrylat-Trimethylammoniumethylmethacrylat-Terpolymere. Butylmethacrylat-Methylmethacrylat-Dimethylaminoethylmethacrylat-Terpolymere

Die Acrylat-Methacrylat-Copolymere sind näher beschrieben in der Europäischen Pharmacopoeia als Polyacrylate Dispersion 30%, in der USP als Ammonio Methacrylate Copolymer und in JPE als Aminoalkyl-Methacrylate Copolymer E.

Als bevorzugte Komponente c) kommt Polyvinylacetat zum Einsatz. Dieses kann als wässrige Dispersion mit Feststoffgehalten von 10 bis 45 Gew.-% eingesetzt werden. Bevorzugt ist zudem Polyvinylacetat mit einem Molekulargewicht zwischen 100.000 und 1.000.000 Dalton besonders bevorzugt zwischen 200.000 und 800.000 Dalton.

Weiterhin können die Formulierungen als Komponenten d) wasserlösliche Polymere in Mengen von 0 bis 15 Gew.-% enthalten. Geeignete wasserlösliche Polymere sind beispielsweise Polyvinylpyrrolidone, Vinylpyrrolidon-Vinylacetat-Copolymere, Polyvinylalkohole, Polyvinylalkohol-Polyethylenglykol-Pfropfcopolymere, Polyethylenglykole, Ethylenglykol-Propylenglykol-Blockcopolymere, Hydroxypropylmethylcellulose, Hydroxypropylcellulose, Hydroxyethylcellulose, Carragenane, Pektine, Xanthane, Alginate.

Gewünschtenfalls können durch Zusatz von pharmazeutisch üblichen Hilfsstoffen (Komponenten e)) in Mengen von 0 bis 15 Gew.-%, beispielsweise wie Säuerungsmitteln, Puffersubstanzen, Süßstoffen, Aromen, Geschmacksverstärkern und Farbstoffen Geschmack und Aussehen der aus den Formulierungen erhaltenen Tabletten weiter verbessert werden. Folgende Stoffe sind hierbei besonders geeignet: Citronensäure, Weinsäure, Ascorbinsäure, Natriumdihydrogenphosphat, Cyclamat, Saccharin-Na, Aspartam, Menthol, Pfefferminzaroma, Fruchtaromen, Vanillearoma, Glutamat, Riboflavin, Betacarotin, wasserlösliche Farbstoffe, feinteilige Farblacke.

Durch Zusatz von Verdickungsmitteln wie hochmolekularen Polysacchariden kann das Mundgefühl durch Erhöhung der Weichheit und des Volumengefühls zusätzlich verbessert werden.

Weiterhin können als Komponenten e) auch Tenside zugegeben werden. Als Tenside eignen sich beispielsweise Natriumlaurylsulfat, Dioctylsulfosuccinat, alkoxilierte Sorbitanester wie Polysorbat 80, polyalkoxilierte Derivate von Rizinusöl oder hydriertem Rizinusöl, beispielsweise Cremophor® RH 40, alkoxilierte Fettsäuren, alkoxilierte Hydroxyfettsäuren, alkoxilierte Fettalkohole, Alkalisalze von Fettsäuren und Lecithine.

Weiterhin können zur weiteren Verbesserung des Zerfalls auch feinteilige Pigmente zugegeben werden, weil sie die inneren Grenzflächen erhöhen und somit Wasser schneller in die Tablette eindringen kann. Diese Pigmente wie Eisenoxide, Titandioxid, kolloidale oder gefällte Kieselsäure, Calciumcarbonate, Calciumphosphate müssen natürlich sehr feinteilig sein, ansonsten entsteht wiederum ein körniger Geschmack.

Als Wirkstoffe B) können grundsätzlich alle Wirkstoffe eingesetzt werden. Bevorzugt werden die im Folgenden genannten Wirkstoffe, besonders bevorzugt in den genannten Dosierungen, eingesetzt.

Zolmitriptan 2,5 mg, Rizatriptan 5 mg, Diphenhydramin-HCl (geschmacksmaskiert) 20 mg, Brompheniramin 5 mg, Chlorpheniramin 5 mg, Pseudoephedrin (geschmacksmaskiert) 30mg,

Paracetamol (geschmacksmaskiert) 250 mg, Ibuprofen (geschmacksmaskiert) 200 mg, Acetylsalicylsäure 250 mg (geschmacksmaskiert), Hyoscyaminsulfat 0,125 mg, Mirtazapin 15 mg,

Selegelin-HCl 1,25 mg, Ondansetron 4 mg, Olanzapin 5 mg, Clonazepam 1 mg, Cetirizindihydrochlorid 10 mg, Desloratadin 5 mg, Enalaprilmaleat 5 mg, Domperidonmaleat 10 mg, Scopolamin 0,25 mg,

Oxazepam 15 mg, Lorazepam 2,5 mg, Clozapin 25 mg, Dihydroergotaminmesylat 5 mg,

Nicergolin 5 mg, Phloroglucinol 80 mg, Metopimazin 7,5 mg, Triazolam 0,5 mg, Protizolam 0,5 mg,

Tramadol 50 mg, Zolpidemtartrat 5 mg, Cisapride 5 mg, Risperidon 2 mg, Azithromycin 100 mg (geschmacksmaskiert), Roxithromycin 50 mg (geschmacksmaskiert), Clarithromycin 125 mg (geschmacksmaskiert), Erythromycinestolat 250 mg (geschmacksmaskiert),

Apomorphin 20 mg, Fentanyl 0,6 mg,

Es können auch Mischungen von Wirkstoffen eingesetzt werden. Insbesonders geeignete Mischungen sind: Paracetamol oder Ibuprofen oder Acetylsalicylsäure und Pseudoephedrin, Paracetamol oder Ibuprofen oder Acetylsalicylsäure und Diphenhydramin oder Chlorpheniramin oder Brompheniramin oder Loratadin.

Die genannten Dosierungen stellen die Absolutmengen des jeweiligen Wirkstoffs pro Arzneiform dar. Die Konzentration des Hilfsstoffsanteils und des Wirkstoffanteils in der fertigen Arzneiform richtet sich nach der Größe der Arzneiform. Bei Tabletten sind übliche Tablettengewichte: 50 bis 1000mg bevorzugt 80 bis 600mg

Die Wirkstoffe können auch mit einem üblichen geschmacksmaskierenden Überzug versehen sein. Geeignete Polymere für solche Coatings sind: Aminoalkyl methacrylate Copolymer E (Eudragit E oder EPO), Polyvinylalkohole in verschiedenen Formulierungen (Opadry AMB, Kollicoat Protect), Kombinationen von wasserunlöslichen Polymeren wie z. B. Polyvinylacetat, Poly(meth)acrylate (Eudragit NE 30 D, NM 30D, RL, RS, RD, Kollicoat EMM 30 D), Ethylcellulose mit wasserlöslichen oder wasserquellbaren nieder- oder hochmolekularen Stoffen (Povidon, Copovidon, HPMC, HPC, Polyethylenglykole, Poloxamere, Polyethylenglykol-Polyvinylalkohol-Pfropfcopolymere, Zucker, Zuckeralkohole, organischen oder anorganische Salze), Kombinationen von wasserlöslichen Filmbildnern (Polyethylenglykol-Polyvinylalkohol-Pfropfcopolymere, HPMC, Polyvinylalkohole) mit Fetten, Wachsen, Fettsäuren und Fettalkoholen.

Die Herstellung der erfindungsgemäßen Formulierungen kann durch Aufbau-Agglomeration in Mischern, Wirbelschichtgeräten oder Sprühtürmen erfolgen. Dabei werden feste Ausgangsmaterialien und Granulierflüssigkeit zunächst miteinander vermischt und das feuchte Mischgut anschliessend getrocknet. Gemäß der vorliegenden Erfindung wird als Granulierflüssigkeit eine wässrige Dispersion der Komponente c), des wasserunlöslichen Polymers, eingesetzt.

Gemäß einer Ausführungsform der Erfindung werden ein oder mehrere Wirkstoffe zusammen mit der Zucker oder Zuckeralkohol, Sprengmittel und gewünschtenfalls den Komponenten d) und e) in der Wirbelschicht vorgelegt.

Bei der Agglomeration in der Wirbelschicht wird eine wässrige Dispersion des wasserunlöslichen Polymers (Komponente c)) auf eine wirbelnde Mischung aus Zucker oder Zuckeralkohol, Sprengmittel, Wirkstoffen und gegebenenfalls weiteren Komponenten d) und e) gesprüht, wodurch die feinen Teilchen agglomerieren. Die Zulufttemperaturen betragen 30 bis 100°C, die Ablufttemperaturen 20 bis 70°C.

Insbesondere können bei dieser Agglomeration als weitere Komponente e) die folgenden Hilfsstoffe eingearbeitet werden:

Farbstoffe, Süßstoffe , Aromen, weitere Sprengmittel, Carbonate, Bicarbonate, Säuerungsmittel oder weitere Hilfsstoffe. Die Verwendung von Farbstoffen, wobei anorganische Pigmente, Organische Farblacke oder wasserlösliche Farbstoffe verwendet werden können, führt beispielsweise zu einheitlich durchgefärbten, schnellzerfallenden Tabletten. Geeignete Farbstoffe sind z. B. Riboflavin, Betacarotin, Anthocyane, Carmin, Indigocarmin, Gelborange S, Chinolingelb, Indigotinlack, Brilliant Blau, Sunset Yellow. Diese weiteren Stoffe können entweder in fester Form in die Wirbelbett-Vorlage gegeben werden oder in der Dispersion der komponenten c) gelöst oder dispergiert werden. Falls die Dispersion unverträglich mit einem solchen Stoff ist, kann dieser in Lösung oder als Suspension auch vor oder nach der Agglomeration mit der Dispersion der Komponenten c) aufgesprüht werden.

Bei der Herstellung in Sprühtürmen wird vorzugsweise die sogenannte FSD- oder SBD-Technologie (FSD: Fluidized spray drying; SBD: Spray bed drying) eingesetzt. Hierbei wird eine Lösung des Zuckers oder Zuckeralkohols in Wasser zunächst sprühgetrocknet, im unteren Teil des Sprühtrockners oder in einem angeschlossenen Wirbelbett erfolgt die Zugabe von Sprengmittel und die Eindüsung einer wässrigen Dispersion des wasserunlöslichen Polymeren, wodurch die Teilchen agglomerieren. In einer besonderen Variante wird das Sprengmittel in der Lösung des Zuckers oder Zuckeralkohols in Wasser dispergiert und der Prozeß wie oben beschrieben durchgeführt. Feine Teilchen können ferner nochmals vor die Sprühdüse der Zucker oder Zuckeralkohollösung geblasen werden und zusätzlich agglomeriert werden. Es ist im Sprühturm, FSD oder SBD auch eine Prozeßführung ausgehend von der kristallinen Form des Zuckers oder Zuckeralkohols möglich. Dabei wird der kristalline Zucker oder Zuckeralkohol am Kopf des Sprühturms oder in den Feingut-Recyclingstrom zugegeben. Durch das Versprühen einer wässrigen Dispersion des wasserunlöslichen Polymeren wird dieser kristalline Feststoff im Turm agglomeriert.

Für den Agglomerationsprozess kann es sich als günstig erweisen, einen mehrstufigen Sprühprozess zu fahren. Zu Anfang wird die Sprührate niedrig gehalten, um ein Über feuchten der Produktvorlage und damit deren Verkleben zu verhindern. Mit zunehmender Prozessdauer kann die Sprührate erhöht und damit die Agglomerationstendenz erhöht werden. Es ist ebenfalls möglich die Zuluftmenge und/oder -temperatur in entsprechender Weise während des Prozesses anzupassen. Besonders während der Trocknungsphase ist es vorteilhaft, die Zuluftmenge zu reduzieren und damit einem Abrieb der Agglomerate durch eine hohe mechanischen Belastung vorzubeugen.

Als weitere Anpassungsparameter für die Agglomeratgröße kann die Feinheit des Sprühtröpfchens der Bindemittellösung bzw. -dispersion (einstellbar über den Zerstäubungsgasdruck), die Düsengeometrie und Abstand der Düse zum Produktbett angesehen werden. Je feiner und einheitlicher gesprüht wird, desto feiner und einheitlicher resultieren die Agglomerate. Je weiter die Düse vom Produktbett entfernt ist, desto schlechter ist das Agglomerationsverhalten.

Weiterhin kann die Agglomeration auch in einem Mischer durch eine kontinuierlich geführte Mischaggregation erfolgen. Eine solche kontinuierlich geführte Form der Mischaggregation ist die sogenannte "Schugi-Granulation". Dabei werden in einem kontinuierlich arbeitenden vertikal angeordneten Hochgeschwindigkeitsmischer feste Ausgangsmaterialien und die das wasserunlösliche Polymer enthaltende Granulierflüssigkeit intensiv miteinander vermischt (siehe auch M. Bohnet, "Mechanische Verfahrenstechnik", Wiley VCH Verlag, Weinheim 2004, S. 198 ff.)

Gemäß einer besonderen Ausführungsform wird das Sprengmittel in der wässrigen Dispersion des wasserunlöslichen Polymers suspendiert.

Die so erhaltenen Agglomerate weisen eine mittlere Teilchengröße von 100 - 600 $\mu$m, bevorzugt 120 - 500 $\mu$m und besonders bevorzugt von 140 - 400 $\mu$m auf.

Das wasserunlösliche, filmbildende Polymer dient dabei als Agglomerationsmittel, um die feinen Zucker oder Zuckeralkoholkristalle, die Wirkstoffpartikel und das Sprengmittel sowie gebenenfalls weitere Hilfsstoffe zu größeren Teilchen zu agglomerieren.

Gemäß einer weiteren Ausführungsform der Erfindung kann auch zunächst der Hilfsstoffanteil A) agglomeriert werden und anschliessend in einem weiteren Granulationsschritt eine Agglomeration mit einem oder mehreren Wirkstoffen erfolgen.

Gemäß einer Variante dieser Ausführungsform werden der agglomerierte Hilfsstoffanteil und Wirkstoff in der Wirbelschicht vorgelegt und mittels einer Bindemittellösung granuliert. Die Bindemittellösung enthält als Bindemittel dabei ein wasserlösliches Polymer ausgewählt aus der Gruppe der Komponenten d). Es handelt sich bevorzugt um eine wässrige Lösung. Die Bindemittelkonzentration kann 1 bis 40 Gew.-% betragen.

Bevorzugte Bindemittel sind wasserlösliche Polyvinylpyrrolidone mit K-Werten nach Fikentscher von 12 bis 120, insbesondere K30, Vinylpyrrolidon-Vinylacetat-Copolymere aus 30 bis 70 Gew.-% N-Vinypyrrolidon, bevorzugt 40 bis 60 Gew.-%, und 30 bis 70 Gew.-%, bevorzugt 40 bis 60 Gew.-% Vinylacetat, weiterhin Polyvinylalkohole, Pfropfcopolymere aus Polyethylenglykol und Polyvinylalkohol, Hydroxypropylmethylcellulose, Hydroxypropylcellulose, Hydroxyethylcellulose sowie Gelatine.

Gemäß einer zweiten Variante wird der agglomerierte Hilfsstoffanteil in der Wirbelschicht vorgelegt und der Wirkstoffanteil in die oben beschriebene Bindemittellösung eingearbeitet.

Gemäß einer weiteren Variante, bei der das wasserlösliche Bindemittel (Komponente d)) obligatorisch in dem voragglomerierten Hilfsstoffanteil bereits enthalten ist, vorzugsweise in Mengen von 0,2 bis 10 Gew.-%, wird als Granulierflüssigkeit Wasser ohne polymeres Bindemittel eingesetzt. Andere Hilfsstoffe aus der Gruppe der Komponenten e) können dabei dem für die Granulierung verwendeten Wasser zugegeben werden. Weiterhin kann es sich empfehlen einen der genannten Zucker oder Zuckeralkohole dem Wasser zuzugeben.

Für alle diese Varianten der Ausführungsform, bei der zunächst der Hilfsstoffanteil agglomeriert wird, werden ansonsten die gleichen oben beschriebenen Vorrichtungen und Prozessparameter angewandt wie bei der gleichzeitigen Agglomeration von Hilfsstoffanteil und Wirkstoffanteil.

Die erfindungsgemäßen Formulierungen können vorteilhaft auch für die Herstellung von Tabletten verwendet werden, die vor der Anwendung in einem Glas Wasser zerfallen gelassen werden. Auch die Herstellung von Tabletten, die intakt geschluckt werden, ist natürlich möglich.

Für die Herstellung von Tabletten können die üblichen Verfahren verwendet werden, wobei die Direkttablettierung und die Walzenkompaktierung besondere Vorteile bieten. Aufgrund der besonderen Eigenschaften der erfindungsgemäßen Formulierungen werden in der Regel nur Wirkstoff, erfindungsgemäße Formulierung und ein Schmiermittel benötigt. Die Tablettenrezeptur ist somit sehr einfach, sehr reproduzierbar und das Verfahren leicht zu validieren.

Überraschenderweise wurde gefunden, dass ein wasserunlösliches filmbildendes Polymer den Zerfall von Tabletten erheblich beschleunigt. Dies ist umso überraschender, da solche Polymere in der Regel für die Herstellung von retardierten Arzneiformen eingesetzt werden, die innerhalb mehrerer Stunden nicht zerfallen. Die Zerfallszeiten unter Verwendung von Polyvinylacetat als wasserunlöslichem Polymer sind erheblich kürzer als bei wasserlöslichen Polymeren.

Ferner besitzen die erfindungsgemäßen Zubereitungen außergewöhnlich gute Fließfähigkeiten und Verpressbarkeiten, die zu mechanisch sehr stabilen Tabletten führen. Die Bruchfestigkeit der mit Hilfe der erfindungsgemäßen pharmazeutischen Formulierungen erzeugten Tabletten beträgt > 40 N. Häufig liegen die Bruchfestigkeiten oberhalb von 60 N, selbst unter Verwendung schwer pressbarer Wirkstoffe. Die Friabilitäten betragen < 0,2 %. Beschädigungen beim üblichen Tablettenhandling treten somit nicht auf.

Aufgrund des feinen quervernetzten Polyvinylpyrrolidons zeigen die Tabletten nahezu keine Veränderungen der Tablettenoberfläche bei feuchter Lagerung. Im Gegensatz zu grobem quervernetzten Polyvinylpyrrolidon findet sich keine Pickelbildung durch stark aufgequollene Teilchen. Die erfindungsgemäßen Formulierungen sind damit bei Lagerung sehr stabil und behalten ihr ansprechendes Aussehen.

Durch die Granulation werden die Wirkstoffteilchen fest mit den Hilfsstoffteilchen verbunden und es ist keine Entmischung mehr möglich. Dadurch verbessert sich die Gehaltseinheitlichkeit von Tabletten insbesondere von Tabletten mit niedrigem Wirkstoffgehalt erheblich. Die Reproduzierbarkeit der Tabletteneigenschaften ist erheblich verbessert. Auf diese Weise können Wirkstoffe mit im Vergleich zu den Hilfsstoffen sehr unterschiedlichen physiko-chemischen Eigenschaften verarbeitet werden.

Überraschenderweise führt die Granulation zusammen mit Wirkstoffen nicht zu einer Verlangsamung der Zerfallsgeschwindigkeit, obwohl dies durch die stärkere Verklebung der Teilchen zu erwarten wäre.

Die pharmazeutischen Zubereitungen können auch in anderen Arzneiformen Anwendung finden: weitere Arzneiformen sind beispielsweise Sachets oder Granulatbehälter mit Dosiereinrichtung.

Beispiele

Die Herstellung erfolgte durch einen zweistufigen Agglomerationsprozess, wobei zunächst eine geringere Sprührate gewählt und dann die Sprührate erhöht wurde. Folgende Herstellungsbedingungen wurden in einem zweistufigen Agglomerationsprozess angewendet:

| | |
|---|---|
| Ansatzgröße: | 0,6 kg |
| Konzentration der wässrigen Bindemittellösung/-dispersion: | 20 Gew.-% |
| Zulufttemperatur: | 50 °C |
| Ablufttemperatur: | 30 °C |
| Sprührate zu Beginn (10min): | 15 g/min |
| Sprührate nach Umstellen: | 20 g/min |

Als Sprengmittel wurde verwendet: Kollidon CL-SF, Fa. BASF: Crospovidon, quervernetztes Polyvinylpyrrolidon mit einer mittleren Teilchengröße von 17μm.

Als Polyvinylacetat wurde verwendet: Kollicoat SR 30 D, Fa. BASF: kommerziell erhältliche wässrige Polyvinylacetat-Dispersion, stabilisiert mit 2,7 Gew.-% Polyvinylpyrrolidon und 0,3 Gew.-% Natriumdodecylsulfat

Kollicoat® IR, Fa. BASF: kommerziell erhältliches Pfropfpolymer aus 75 gew.-% Polyvinylalkohol und 25 Gew.-% Polyethylenglykol, mittl. Mw. 45.000 D

Kollidon® VA 64, FA. BASF: Copovidon, Copolymer aus 60 Gew.- NVP und 40 Gew.-% VAc

Tabelle 1: Zusammensetzung der Agglomerate A bis E in Gew.-%

| | A | B | C | D | E |
|---|---|---|---|---|---|
| Mannitol ($d_{0,5}$:36μm) | 89,32 | 73,0 | 83,2 | 86,6 | 89,32 |
| Kollidon® CL-SF | 5,00 | 5,0 | 5,0 | 5,0 | 5,00 |
| Kollicoat® SR 30 D (Feststoff) | 5,00 | 5,0 | 5,0 | 5,0 | 5,00 |
| Loperamid-HCl | 0,68 | - | - | - | - |
| Coffein (feines Pulver) | - | 17,0 | - | - | - |
| Piroxicam | - | - | - | 3,4 | - |
| Famotidin | - | - | 6,8 | - | - |
| Risperidon | - | - | - | - | 0,68 |

Die so hergestellten Agglomerate wurden mit 2,0 Gew.-% Schmiermittel (Mg-Stearat) in einem Turbulamischer für 10 min gemischt. Anschließend wurden diese Mischungen auf einer voll instrumentierten Exzenterpresse (Korsch XP 1) zu biplanen Tabletten mit einem Gewicht von 300mg, einem Durchmesser von 10mm und einer Bruchfestigkeit von 40-55N verpresst.

Die Tabletten wurden hinsichtlich Bruchfestigkeit (Tablettentester HT-TMB-Cl-12 F, Fa. Kraemer), Zerfallszeit in Phosphatpuffer pH 7,2 (Zerfallstester ZT 74, Erweka) und Freisetzungsgeschwindigkeit in künstlichem Magensaft pH 1,2 (Freisetzungsgerät, Erweka) untersucht.

Tabelle 2: Tabletteneigenschaften Formulierungen A bis E

| | Tablettierdaten | Tablettenparameter | | |
|---|---|---|---|---|
| | Presskraft [kN] | Bruchfestigkeit [N] | Zerfallszeit [s] | Freisetzungsgeschwindigkeit [% nach 10min] |
| A | 3,5 | 46 | 18 | 70 |
| B | 3,2 | 48 | 23 | 100 |
| C | 4,3 | 51 | 37 | 100 |
| D | 4,1 | 42 | 35 | 60 |
| E | 3,9 | 56 | 27 | 100 |

Granulation des voragglomerierten Hilfsstoffsanteils mit Wirkstoff

Der schnell zerfallende Hilfsstoff wurde durch Agglomeration in der Wirbelschicht von Mannitol (90 Gew.-%) und quervernetztem PVP (5 Gew.-%) mit Polyvinylacetat (5 Gew.-%) hergestellt. Das so hergestellte Produkt wurde einem weiteren Granulationsschritt unterzogen, wobei im ersten Versuch jeweils voragglomerierter Hilfsstoff und Wirkstoff vorgelegt und mittels Bindemittellösung granuliert wurden, bei der zweiten Variante wurde nur voragglomerierter Hilfsstoff vorgelegt, der Wirkstoff wurde in die Bindemittellösung eingearbeitet.

Als Granulationsverfahren wurden sowohl das Wirbelschichtverfahren als auch die Mischergranulation eingesetzt. Prinzipiell sind alle Granulationverfahren anwendbar. Das Bindemittel kann auch trocken untergemischt werden und anschließend Wasser aufgesprüht bzw. zugegeben werden.

In einer besonderen Ausführung wurde der schnellzerfallende Hilfsstoff mit dem Wirkstoff nur durch Zugabe von Wasser granuliert. Die Granulierwirkung ließ sich hierbei verbessern, indem dem Wasser ein Zucker oder Zuckeralkohol zugesetzt wurden.

Ähnlich wie unter Variante 1 beschrieben können auch bei dieser Granulation Farbstoffe, Süßstoffe, Aromen, weitere Sprengmittel, Carbonate, Bicarbonate, Säuerungsmittel oder weitere Hilfsstoffe verwendet werden.

Tabelle 3: Prozessparameter

| | Wirbelschichtgranulation | | Mischergranulation |
|---|---|---|---|
| | F | G | H/J |
| Ansatzgröße [g] | 600 | 600 | 1200 |
| Zulufttemperatur [°C] | 50 | 55 | - |
| Ablufttemperatur [°C] | 30 | 30 | - |
| Sprührate [g/min] | 17 | 20 | 30 |
| Mischer [Upm] | - | - | 200 |
| Zerhacker [Upm] | - | - | 400 |
| Granulierzeit [min] | - | - | 10 |
| Bemerkungen | Granulation mit Bindemittellösung | Wirkstoff ist in der Bindemittellösung eingearbeitet | Wirkstoff ist in der Bindemittellösung eingearbeitet (Versuch J) |

Tabelle 4: Zusammensetzung der Agglomerate F bis J in Gew.-%

| | Wirbelschichtgranulation | | Mischergranulation | |
|---|---|---|---|---|
| | F | G | H | J |
| Mannitol ($d_{0,5}$:36$\mu$m) | 86,64 | 71,94 | 71,94 | 86,64 |
| Kollidon CL-SF | 4,81 | 4,00 | 4,00 | 4,81 |
| Kollicoat SR 30 D (Feststoff) | 4,81 | 4,00 | 4,00 | 4,81 |
| Loperamid-HCl | 0,68 | - | - | 0,68 |
| Coffein (feines Pulver) | - | 17,00 | 17,00 | - |
| Kollidon 30 | - | - | - | 3,06 |
| Kollicoat® IR | 3,06 | - | 3,06 | - |
| Kollidon® VA 64 | - | 3,06 | - | - |

Die aus der Mischergranulation resultierenden Granulate wurden nach der Granulation durch ein 1 mm-Sieb gesiebt, über Nacht bei Raumtemperatur auf einer Horde getrocknet und anschließend über ein 0,8mm-Sieb gegeben.
Die so hergestellten Agglomerate wurden mit 2,0 Gew.-% Schmiermittel (Mg-Stearat) und in den Beispielen F und J noch mit zusätzlichen 3% Kollidon CL-SF abgemischt und anschließend auf einer Rundläufertablettenpresse (Korsch XL 100) zu Tabletten mit 40-50N Bruchfestigkeit verpresst.
Die Tabletten wurden hinsichtlich Bruchfestigkeit (Tablettentester HT-TMB-Cl-12 F, Fa. Kraemer), Zerfallszeit in Phosphatpuffer pH 7,2 (Zerfallstester ZT 74, Erweka) und Freisetzungsgeschwindigkeit in künstlichem Magensaft bei pH 1,2 (Freisetzungsgerät, Erweka) untersucht.

Tabelle 5: Tabletteneigenschaften Formulierungen F bis J

| | Bruchfestigkeit [N] | Friabilität [%] | Zerfallszeit [s] | Freisetzungsgeschwindigkeit [% nach 10min] |
|---|---|---|---|---|
| F | 43 | <0,2 | 89 | 60 |
| G | 48 | <0,2 | 62 | 95 |
| H | 42 | <0,2 | 51 | 100 |
| J | 45 | <0,2 | 59 | 70 |

**Patentansprüche**

1. Pharmazeutische Formulierung in Form von Agglomeraten enthaltend

   A) einen Hilfsstoffanteil aus

      a) 60 - 97 Gew.-% Trehalose, Mannit, Erythrit, Isomalt, Maltit, Lactit, Xylit, Sorbit oder Mischungen davon,
      b) 1 - 25 Gew.-% eines Sprengmittels
      c) 1 - 15 Gew.-% wasserunlösliche, filmbildende Polymere
      d) 0 - 15 Gew.-% wasserlösliche Polymere, und
      e) 0 - 15 Gew.-% weitere pharmazeutisch übliche Hilfsstoffe

   wobei die Summe der Komponenten a) bis e) 100 Gew.-% beträgt,
   und
   B) mindestens einen Wirkstoff.

2. Formulierung nach Anspruch 1 enthaltend

   A) einen Hilfsstoffanteil aus

      a) 60 - 97 Gew.-% Trehalose, Mannit, Erythrit, Isomalt, Maltit, Lactit, Xylit, Sorbit oder Mischungen davon,
      b) 1 - 25 Gew.-% eines Sprengmittels ausgewählt aus der Gruppe bestehend aus Crospovidon, Croscarmellose, Natriumcarboxymethylstärke und L-Hydroxypropylcellulose,
      c) 1 - 15 Gew.-% wasserunlösliche, filmbildende Polymere
      d) 0 - 15 Gew.-% wasserlösliche Polymere, und
      e) 0 - 15 Gew.-% weitere pharmazeutisch übliche Hilfsstoffe

   wobei die Summe der Komponenten a) bis e) 100 Gew.-% beträgt,
   und
   B) mindestens einen Wirkstoff.

3. Formulierung nach Anspruch 1 oder 2, wobei die die mittlere Teilchengröße der Agglomerate 100 $\mu$m bis 600 $\mu$m beträgt.

4. Formulierung nach einem der Ansprüche 1 bis 3, enthaltend als Zuckeralkohol Mannit oder, Erythrit oder Mischungen davon.

5. Formulierung nach einem der Ansprüche 1 bis 4, enthaltend ein Croscarmellose als Natrium- oder Calcium-Salz.

6. Formulierung nach einem der Ansprüche 1 bis 5, enthaltend eine L-Hydroxypropylcellulose mit 5 bis 16% Hydroxypropoxy-Gruppen

7. Formulierung nach einem der Ansprüche 1 bis 6, enthaltend als Sprengmittel Crospovidon

8. Formulierung nach einem der Ansprüche 1 bis 7 , enthaltend als wasserunlösliches filmbildendes Polymer Polyvinylacetat.

9. Formulierung nach einem der Ansprüche 1 bis 8 wobei das wasserunlösliche filmbildende Polymer Polyvinylacetat in Form einer wässrigen Dispersion eingesetzt wird.

10. Formulierung nach einem der Ansprüche 1 bis 9 , wobei als wasserlösliches Polymer Polyvinylpyrrolidon verwendet wird.

11. Formulierung nach einem der Ansprüche 1 bis 10, wobei als weitere pharmazeutisch übliche Stoffe Säuerungsmittel, Süßstoffe, Aromen, Geschmacksverstärker, Farbstoffe, Verdickungsmittel, Tenside und feinteilige Pigmente verwendet werden.

12. Formulierung nach einem der Ansprüche 1 bis 11, enthaltend Agglomerate mit einem Hilfsstoffanteil A) aus

a) 70 - 95 Gew.-% Trehalose, Mannit, Erythrit, Isomalt, Maltit, Lactit, Xylit, Sorbit oder Mischungen davon
b) 2 - 15 Gew.-% eines Sprengmittels,
c) 1 - 10 Gew.-% wasserunlösliche, filmbildende Polymere
d) 0 - 10 Gew.-% wasserlösliches Polyvinylpyrrolidon, und
e) 0 - 15 Gew.-% weitere pharmazeutisch übliche Hilfsstoffe.

13. Formulierung nach einem der Ansprüche 1 bis 12, enthaltend Agglomerate mit einem Hilfsstoffanteil A) aus

a) 75 - 95 Gew.-% Mannit oder Erythrit oder einer Mischung davon,
b) 3 - 10 Gew.-% eines Sprengmittels,
c) 1- 10 Gew.-% Polyvinylacetat,
d) 0 - 5 Gew.-% wasserlösliches Polyvinylpyrrolidon, und
e) 0 - 15 Gew.-% weitere pharmazeutisch übliche Hilfsstoffe.

14. Tabletten, erhalten unter Verwendung einer pharmazeutischen Formulierung nach einem der Ansprüche 1 bis 13, wobei die Tabletten in wässrigem Milieu Zerfallszeiten von kleiner 60 Sekunden aufweisen.

15. Tabletten nach Anspruch 14, wobei die Tabletten eine Bruchfestigkeit größer 40 N aufweisen.

16. Tabletten nach Anspruch 14 oder 15, enthaltend 20 bis 99 Gew.-%, bezogen auf das gesamte Tablettengewicht, einer pharmazeutischen Formulierung gemäß einem der Ansprüche 1 bis 13.

17. Tabletten nach einem der Ansprüche 14 bis 16, enthaltend weitere Hilfsstoffe.

18. Verfahren zur Herstellung einer pharmazeutischen Formulierung gemäß einem der Ansprüche 1 bis 13, **dadurch gekennzeichnet, dass**

A) ein Hilfsstoffanteil aus

a) 60 - 97 Gew.-% Trehalose, Mannit, Erythrit, Isomalt, Maltit, Lactit, Xylit, Sorbit oder Mischungen davon,
b) 1 - 25 Gew.-% eines Sprengmittels
c) 1 - 15 Gew.-% wasserunlösliche, filmbildende Polymere
d) 0 - 15 Gew.-% wasserlösliche Polymere, und
e) 0 - 15 Gew.-% weitere pharmazeutisch übliche Hilfsstoffe

wobei die Summe der Komponenten a) bis e) 100 Gew.-% beträgt,
und B) mindestens ein Wirkstoff,
in Gegenwart von Wasser agglomeriert werden.

19. Verfahren nach Anspruch 18, **dadurch gekennzeichnet, dass** Trehalose, Mannit, Erythrit, Isomalt, Maltit, Lactit, Xylit, Sorbit oder Mischungen davon a), Sprengmittel b)und Wirkstoff B) mit einer wässrigen Dispersion des wasserunlöslichen Polymers c) agglomeriert werden.

20. Verfahren nach Anspruch 18 oder 19, **dadurch gekennzeichnet, dass** die wässrige Dispersion des wasserunlöslichen Polymers zusätzlich Sprengmittel suspendiert enthält.

21. Verfahren nach Anspruch 18, **dadurch gekennzeichnet, dass** in einem ersten schritt der Hilfsstoffanteil A) agglomeriert wird und die entstehenden Agglomerate in einem zweiten Schritt mit dem Wirkstoff B) agglomeriert werden.

22. Verfahren nach einem der Ansprüche 18 bis 21, **dadurch gekennzeichnet, dass** die Agglomeration in einem Wirbelschichtgranulator, einem Mischer oder einem Sprühturm erfolgt.

**Claims**

1. A pharmaceutical formulation in the form of agglomerates comprising

A) an excipient content composed of

a) 60-97% by weight of trehalose, mannitol, erythritol, isomalt, maltitol, lactitol, xylitol, sorbitol or mixtures thereof,
b) 1-25% by weight of a disintegrant,
c) 1-15% by weight of water-insoluble, film-forming polymers
d) 0-15% by weight of water-soluble polymers and
e) 0-15% by weight of further pharmaceutically customary excipients

the total of the components a) to e) being 100% by weight,
and
B) at least one active ingredient.

2. The formulation according to claim 1, comprising

A) an excipient content composed of

a) 60-97% by weight of trehalose, mannitol, erythritol, isomalt, maltitol, lactitol, xylitol, sorbitol or mixtures thereof,
b) 1-25% by weight of a disintegrant, selected from the group consisting of crospovidone, croscarmellose, sodium carboxymethylstarch and L-hydroxypropylcellulose,
c) 1-15% by weight of water-insoluble, film-forming polymers
d) 0-15% by weight of water-soluble polymers and
e) 0-15% by weight of further pharmaceutically customary excipients

the total of the components a) to e) being 100% by weight,
and
B) at least one active ingredient.

3. The formulation according to claim 1 or 2, where the average particle size of the agglomerates is from 100 $\mu$m to 600 $\mu$m.

4. The formulation according to any of claims 1 to 3, comprising mannitol or erythritol or mixtures thereof as sugar alcohol.

5. The formulation according to any of claims 1 to 4, comprising croscarmellose as sodium or calcium salt.

6. The formulation according to any of claims 1 to 5, comprising an L-hydroxypropylcellulose having 5 to 16% hydroxypropoxy groups.

7. The formulation according to any of claims 1 to 6, comprising crospovidone as disintegrant.

8. The formulation according to any of claims 1 to 7, comprising polyvinyl acetate as water-insoluble film-forming polymer.

9. The formulation according to any of claims 1 to 8, where the water-insoluble film-forming polymer polyvinyl acetate is employed in the form of an aqueous dispersion.

10. The formulation according to any of claims 1 to 9, where polyvinylpyrrolidone is used as water-soluble polymer.

11. The formulation according to any of claims 1 to 10, where acidifiers, sweeteners, flavors, flavor enhancers, colorants, thickeners, surfactants and finely divided pigments are used as further pharmaceutically customary substances.

12. The formulation according to any of claims 1 to 11, comprising agglomerates with an excipient content A) composed of

a) 70-95% by weight of trehalose, mannitol, erythritol, isomalt, maltitol, lactitol, xylitol, sorbitol or mixtures thereof,
b) 2-15% by weight of a disintegrant,
c) 1-10% by weight of water-insoluble, film-forming polymers,
d) 0-10% by weight of water-soluble polyvinylpyrrolidone, and
e) 0-15% by weight of further pharmaceutically customary excipients.

13. The formulation according to any of claims 1 to 12, comprising agglomerates with an excipient content A) composed of

a) 75-95% by weight of mannitol or erythritol or a mixture thereof,
b) 3-10% by weight of a disintegrant,
c) 1-10% by weight of polyvinyl acetate,
d) 0-5% by weight of water-soluble polyvinylpyrrolidone, and
e) 0-15% by weight of further pharmaceutically customary excipients.

14. A tablet obtained using a pharmaceutical formulation according to any of claims 1 to 13, where the tablet has a disintegration time of less than 60 seconds.

15. The tablet according to claim 14, where the tablet has a hardness greater than 40 N.

16. The tablet according to claim 14 or 15, comprising from 20 to 99% by weight, based on the total tablet weight, of a pharmaceutical formulation according to any of claims 1 to 13.

17. The tablet according to any of claims 14 to 16, comprising further excipients.

18. A process for producing a pharmaceutical formulation according to any of claims 1 to 13, which comprises agglomerating

A) an excipient content composed of

a) 60-97% by weight of trehalose, mannitol, erythritol, isomalt, maltitol, lactitol, xylitol, sorbitol or mixtures thereof,
b) 1-25% by weight of a disintegrant
c) 1-15% by weight of water-insoluble, film-forming polymers
d) 0-15% by weight of water-soluble polymers and
e) 0-15% by weight of further pharmaceutically customary excipients

the total of the components a) to e) being 100% by weight,
and B) at least one active ingredient,
in the presence of water.

19. The process according to claim 18, wherein trehalose, mannitol, erythritol, isomalt, maltitol, lactitol, xylitol, sorbitol or mixtures thereof a), disintegrant b) and active ingredient B) are agglomerated with an aqueous dispersion of the water-insoluble polymer c).

20. The process according to claim 18 or 19, wherein the aqueous dispersion of the water-insoluble polymer additionally comprises suspended disintegrant.

21. The process according to claim 18, wherein the excipient content A) is agglomerated in a first step, and the resulting agglomerates are agglomerated with the active ingredient B) in a second step.

22. The process according to any of claims 18 to 21, wherein the agglomeration takes place in a fluidized bed granulator, a mixer or a spray tower.

**Revendications**

1. Formulation pharmaceutique sous forme d'agglomérats contenant

A) une proportion d'adjuvant constituée de

a) 60-97% en poids de tréhalose, de mannitol, d'érythritol, d' isomalt, de maltitol, de lactitol, de xylitol, de sorbitol ou des mélanges de ceux-ci,
b) 1-25% en poids d'un agent de désintégration,
c) 1-15% en poids de polymères filmogènes insolubles dans l'eau

d) 0-15% en poids de polymères solubles dans l'eau, et

e) 0-15% en poids d'autres adjuvants pharmaceutiquement usuels,

la somme des composants a) à e) valant 100% en poids,

et

B) au moins une substance active.

2. Formulation selon la revendication 1, contenant

    A) une proportion d'adjuvant constituée de

        a) 60-97% en poids de tréhalose, de mannitol, d'érythritol, d'isomalt, de maltitol, de lactitol, de xylitol, de sorbitol ou des mélanges de ceux-ci

        b) 1-25% en poids d'un agent de désintégration, choisi dans le groupe constitué par la crospovidone, la croscarmellose, le carboxyméthylamidon de sodium et la L-hydroxypropylcellulose,

        c) 1-15% en poids de polymères filmogènes insolubles dans l'eau

        d) 0-15% en poids de polymères solubles dans l'eau, et

        e) 0-15% en poids d'autres adjuvants pharmaceutiquement usuels,

    la somme des composants a) à e) valant 100% en poids

    et

    B) au moins une substance active.

3. Formulation selon la revendication 1 ou 2, dans laquelle la grosseur moyenne des agglomérats est de 100 $\mu$m à 600 $\mu$m.

4. Formulation selon l'une quelconque des revendications 1 à 3, contenant, comme alcool de sucre, du mannitol ou de l'érythritol ou des mélanges de ceux-ci.

5. Formulation selon l'une quelconque des revendications 1 à 4, contenant une croscarmellose sous forme de sel sodique ou calcique.

6. Formulation selon l'une quelconque des revendications 1 à 5, contenant une L-hydroxypropylcellulose présentant 5 à 16% de groupes hydroxypropoxy.

7. Formulation selon l'une quelconque des revendications 1 à 6, contenant de la crospovidone comme agent de désintégration.

8. Formulation selon l'une quelconque des revendications 1 à 7, comme polymère filmogène insoluble dans l'eau, contenant, du poly(acétate de vinyle).

9. Formulation selon l'une quelconque des revendications 1 à 8, le polymère filmogène insoluble dans l'eau poly(acétate de vinyle) étant utilisé sous forme d'une dispersion aqueuse.

10. Formulation selon l'une quelconque des revendications 1 à 9, de la polyvinylpyrrolidone étant utilisée comme polymère soluble dans l'eau.

11. Formulation selon l'une quelconque des revendications 1 à 10, des agents acidifiants, des édulcorants, des arômes, des exhausteurs de goût, des colorants, des épaississants, des agents tensioactifs et des pigments finement divisés étant utilisés comme autres substances pharmaceutiquement usuelles.

12. Formulation selon l'une quelconque des revendications 1 à 11, contenant des agglomérats avec une proportion d'adjuvant A) constituée de

    a) 70-95% en poids de tréhalose, de mannitol, d'érythritol, d' isomalt, de maltitol, de lactitol, de xylitol, de sorbitol ou des mélanges de ceux-ci,

    b) 2-15% en poids d'un agent de désintégration,

    c) 1-10% en poids de polymères filmogènes insolubles dans l'eau

d) 0-10% en poids de polyvinylpyrrolidone soluble dans l'eau, et
e) 0-15% en poids d'autres adjuvants pharmaceutiquement usuels.

13. Formulation selon l'une quelconque des revendications 1 à 12, contenant des agglomérats avec une proprtion d'aduvant A) constituée de

a) 75-95% en poids de mannitol ou d'érythritol ou d'un mélange de ceux-ci,
b) 3-10% en poids d'un agent de désintégration,
c) 1-10% en poids de poly(acétate de vinyle),
d) 0-5% en poids de polyvinylpyrrolidone soluble dans l'eau, et
e) 0-15% en poids d'autres adjuvants pharmaceutiquement usuels.

14. Comprimés obtenus par l'utilisation d'une formulation pharmaceutique selon l'une quelconque des revendications 1 à 13, les comprimés présentant des temps de décomposition en milieu aqueux inférieurs à 60 secondes.

15. Comprimés selon la revendication 14, les comprimés présentant une résistance à la rupture supérieure à 40 N.

16. Comprimés selon la revendication 14 ou 15, contenant 20 à 99% en poids, par rapport au poids total du comprimé, d'une formulation pharmaceutique selon l'une quelconque des revendications 1 à 13.

17. Comprimés selon l'une quelconque des revendications 14 à 16, contenant d'autres adjuvants.

18. Procédé pour la préparation d'une formulation pharmaceutique selon l'une quelconque des revendications 1 à 13, **caractérisé en ce qu'**on agglomère

A) une proportion d'adjuvant constituée de

a) 60-97% en poids de tréhalose, de mannitol, d'érythritol, d'isomalt, de maltitol, de lactitol, de xylitol, de sorbitol ou des mélanges de ceux-ci
b) 1-25% en poids d'un agent de désintégration,
c) 1-15% en poids de polymères filmogènes insolubles dans l'eau
d) 0-15% en poids de polymères solubles dans l'eau, et
e) 0-15% en poids d'autres adjuvants pharmaceutiquement usuels,

la somme des composants a) à e) valant 100% en poids
et
B) au moins une substance active

en présence d'eau.

19. Procédé selon la revendication 18, **caractérisé en ce qu'**on agglomère le tréhalose, le mannitol, l'érythritol, l'isomalt, le maltitol, le lactitol, le xylitol, le sorbitol ou des mélanges de ceux-ci a) l'agent de désintégration b) et la substance active B) avec une dispersion aqueuse du polymère insoluble dans l'eau c).

20. Procédé selon la revendication 18 ou 19, **caractérisé en ce que** la dispersion aqueuse du polymère insoluble dans l'eau contient en outre de l'agent de désintégration en suspension.

21. Procédé selon la revendication 18, **caractérisé en ce que**, dans une première étape, la proportion d'adjuvant A) est agglomérée et les agglomérats formés sont agglomérés dans une deuxième étape avec la substance active B).

22. Procédé selon l'une quelconque des revendications 18 à 21, **caractérisé en ce que** l'agglomération est réalisée dans un granulateur à lit fluidisé, un mélangeur ou une tour de pulvérisation.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2003051338 A
- US 20020071864 A1
- US 6696085 B2
- EP 0839526 A2
- JP 2004265216 A
- WO 2003041683 A
- US 2005244343 A
- US 2005244492 A

**In der Beschreibung aufgeführte Nicht-Patentliteratur**

- **M. BOHNET.** Mechanische Verfahrenstechnik. Wiley VCH Verlag, 2004, 198 ff